# EUROPEAN PATENT SPECIFICATION

(11) **EP 2 198 040 B1**
(45) Date of publication and mention of the grant of the patent: **15.08.2018**
(21) Application number: 08828425.2
(22) Date of filing: 02.09.2008
(51) Int. Cl.: C12Q 1/08, A61K 51/04

(54) **IN VIVO IMAGING OF MYELIN**
IN-VIVO-ABBILDUNG VON MYELIN
IMAGERIE IN VIVO DE LA MYÉLINE

(30) Priority: 31.08.2007 US 969272 P
(43) Date of publication of application: 23.06.2010
(73) Proprietor: Case Western Reserve University, Cleveland, OH 44106 (US)
(72) Inventor: WANG, Yanming, Beachwood, OH 44122 (US); WU, Chunying, Beachwood OH 44122 (US)
(74) Representative: Müller Fottner Steinecke
(86) International application number: PCT/US2008/075031
(87) International publication number: WO 2009/029936

(56) References cited:
- WO-A2-2009/059239
- US-A1- 2003 218 665
- US-A1- 2005 106 100
- US-A1- 2005 215 565
- US-A1- 2006 039 859
- US-A1- 2006 079 448
- US-A1- 2006 142 294
- US-A1- 2006 247 270
- US-A1- 2006 257 866
- US-A1- 2007 035 238
- WU C ET AL: "A novel fluorecent probe that is brain permeable and selectively binds to myelin", JOURNAL OF HISTOCHEMISTRY AND CYTOCHEMISTRY, HISTOCHEMICAL SOCIETY, NEW YORK, NY, US, vol. 54, no. 9, 1 January 2006 (2006-01-01), pages 997-1004, XP002589274, ISSN: 0022-1554
- STANKOFF BRUNO ET AL: "Imaging of CNS myelin by positron-emission tomography.", PROCEEDINGS OF THE NATIONAL ACADEMY OF SCIENCES OF THE UNITED STATES OF AMERICA 13 JUN 2006 LNKD- PUBMED:16754874, vol. 103, no. 24, 13 June 2006 (2006-06-13), pages 9304-9309, XP002684426, ISSN: 0027-8424
- STYREN S D ET AL: "X-34, a fluorescent derivative of Congo red: A novel histochemical stain for Alzheimer's disease pathology", JOURNAL OF HISTOCHEMISTRY AND CYTOCHEMISTRY 2000 US, vol. 48, no. 9, 2000, pages 1223-1232, XP002684427, ISSN: 0022-1554
- MATSUOKA YASUJI ET AL: "An A.beta. sequestration approach using non-antibody A.beta. binding agents", CURRENT ALZHEIMER RESEARCH, SAIF ZONE, SHARJAH [U.A.] : BENTHAM, AE, vol. 2, no. 2, 1 January 2005 (2005-01-01) , pages 265-268, XP009157301, ISSN: 1567-2050
- NAKAYA, TADAO ET AL: "Plastic scintillators. II. Synthesis of some distyrylbenzene derivatives as wavelength shifters in plastic scintillators", BULLETIN OF THE CHEMICAL SOCIETY OF JAPAN , 39(7), 1547-51 CODEN: BCSJA8; ISSN: 0009-2673, 1966, XP002684428,
- XUE, JIEYOU ET AL: "Calculation of some 1,4-bis[(substituted phenyl)ethenyl]benzene derivatives by the HMO method", GAODENG XUEXIAO HUAXUE XUEBAO , 3(1), 93-7 CODEN: KTHPDM; ISSN: 0251-0790, 1982, XP002684429,
- GIBBS-STRAUSS, SUMMER L. ET AL: "Nerve-highlighting fluorescent contrast agents for image-guided surgery", MOLECULAR IMAGING , 10(2), 91-101 CODEN: MIOMBP; ISSN: 1535-3508, 2011, XP8156846,
- WANG Y ET AL: "In Vivo Quantification of Myelin Changes in the Vertebrate Nervous System", JOURNAL OF NEUROSCIENCE, SOCIETY FOR NEUROSCIENCE, US, vol. 29, no. 46, 18 November 2009 (2009-11-18), pages 14663-14669, XP002613606, ISSN: 0270-6474, DOI: 10.1523/JNEUROSCI.4082-08.2009

## Description

### Technical Field

The present invention relates molecular probes and to methods of their use, and particularly relates to molecular probes that readily enter the brain and selectively localize in the myelinated regions.

### Background of the Invention

Myelin is a specialized membrane that ensheathes neuronal axons, promoting efficient nerve impulse transmission (Morell and Quarles (1999) Basic Neurochemistry: molecular, cellular, and medical aspects. In Siegel GJ, ed. Myelin Formation, Structure, and Biochemistry. Lippincott-Raven Publishers, 79-83). Due to its important biological functions in the normal central nervous system (CNS) and its vulnerability in disease, several techniques have been developed to visualize and characterize myelin histopathology. These can be broadly divided into those based upon antibody immunohistochemistry (IHC) (Horton and Hocking (1997) Cereb. Cortex 7:166-177) and more traditional histochemical procedures. The classic histochemical stains include luxol fast blue MBN (Kluver and Barrera (1953) J Neurosci Methods 153: 135-146; Presnell and Schreibman (1997) Humanson's Animal Tissue Techniques, 5th ed.; Kiernan (1999) Histological and Histochemical Methods: Theory and practice, 3rd ed.; Bancroft and Gamble (2002), Theory and Practice of Histological Techniques, 5 ed. and Sudan Black B (Lison and Dagnelie (1935) Bull. d'Histologie Appliquee 12: 85-91). Traditional chromogenic methods also include the Palweigert method ((Weigert (1884) Fortschr Deutsch Med 2: 190-192, (1885) Fortschr Deutsch Med 3:236-239; Clark and Ward (1934) Stain Technol 54:13-16), the Weil stain (Weil (1928) Arch Neurol Psychiatry 20:392-393; Berube et al. (1965) Stain Technol 40:53-62)), the Loyez method (Cook (1974) Manual of Histological Demonstration Methods, 5th ed.), and a method based on horse serum followed by subsequent reaction with diaminobenzidine (McNally and Peters (1998) J Histochem Cytochem 46:541-545). In addition, modified silver stains including the Gallyas method (Pistorio et al. (2005) J Neurosci Methods 153: 135-146) and Schmued's gold chloride technique (Schmued and Slikker (1999) Brain Res 837:289-297) have also been used as simple, high- resolution histochemical markers of myelin. More recently, fluoromyelin (Kanaan et al. (2005) Am J Physiol Regul Integr Comp Physiol 290:R1105-1114) and NIM (Xiang et al. (2005) J Histochem Cytochem 53:1511-1516) were introduced as novel myelin dyes, which enable quick and selective labeling of myelin in brain tissue sections. Wu et al. (2006) J. Histochem. Cytochem. 54: 997-1004 describes a probe that is brain permeable and binds to myelin. Stankoff et al. (2006) PNAS 103: 9304-9309 relates to imaging of CNS, myelin by positron-emission tomography. Although these myelin-staining techniques are widely used in vitro, none can be applied in vivo due to impermeability of the blood-brain barrier (BBB). The lack of *in vivo* molecular probes has limited the progress of myelin imaging and hindered efficacy evaluation of novel myelin repair therapies during their development.

### Summary of the Invention

The scope of the invention is defined by the claims. Any references in the description to methods of treatment refer to the compounds, pharmaceutical compositions and medicaments of the present invention for use in a method for treatment of the human (or animal) body by therapy (or for diagnosis). The present invention relates to a molecular probe for use in the *in vivo* detection by positron emission tomography of myelination in a subject comprising fluorescent stilbenzene derivative that is less than 700 daltons and has a binding affinity (Kd) to isolated myelin fractions of at least about 100nM and a binding affinity (Kd) of up to about 10µM to isolated non-myelin fractions, the fluorescent stilbenzene derivative having the formula 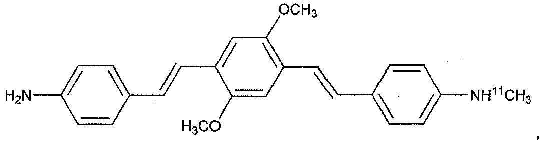 The molecular probe in accordance with the present invention can readily enter the brain following systemic or parenteral administration and bind to myelin membranes.

The present invention also relates to a use of the molecular probe according to the claims in a method of detecting myelination *in vivo* in an animals's brain tissue. The method includes administering *in vivo* to the animal a molecular probe that includes a compound having the formula:

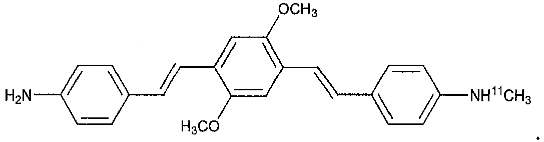

Following administration of the molecular probe, the animal's brain tissue is visualized using an *in vivo* imaging modality.

The present invention further relates to the molecular probe according to the claims for use in a method of detecting a myelin related disorder in a subject. The method includes labeling myelin *in vivo* in the animal's brain tissue by administering to the animal a molecular probe that includes a compound having the formula:

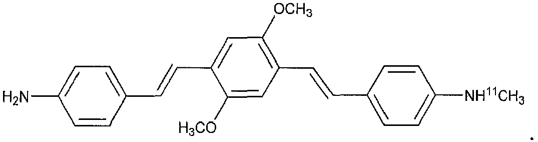

The distribution of the molecular probe in the animal's brain tissue is then visualized. The distribution of the molecular probe can then be correlated with a myelin related disorder in the animal.

The present invention further relates to a use of the molecular probe according to the claims in a method of monitoring the efficacy of a remyelination therapy in an animal. The method includes labeling myelin *in vivo* in the animal's brain tissue with a molecular probe having the formula 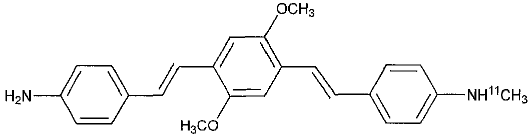 A distribution of the molecular probe in the animal's brain tissue is visualized. The distribution of the molecular probe can then be correlated with the efficacy of the remyelination therapy.

The present invention further relates to use of the molecular probe according to the claims in a method of screening the myelination effects of an agent in an animal. The method includes labeling myelin *in vivo* in the animal's brain tissue with a molecular probe having the formula

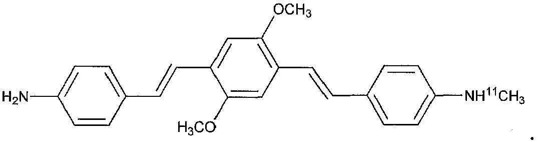

A distribution of the molecular probe in the animal's brain tissue is visualized. The distribution of the molecular probe can then be correlated with the myelination effects of the agent. In one example, the distribution of the molecular probe in the animal's brain tissue can be compared to a distribution of the molecular probe in a control population to determine the efficacy of the agent.

### Brief Description of the Drawings

The foregoing and other features and advantages of the present invention will become apparent to those skilled in the art to which the present invention relates upon reading the following description with reference to the accompanying drawings, in which:

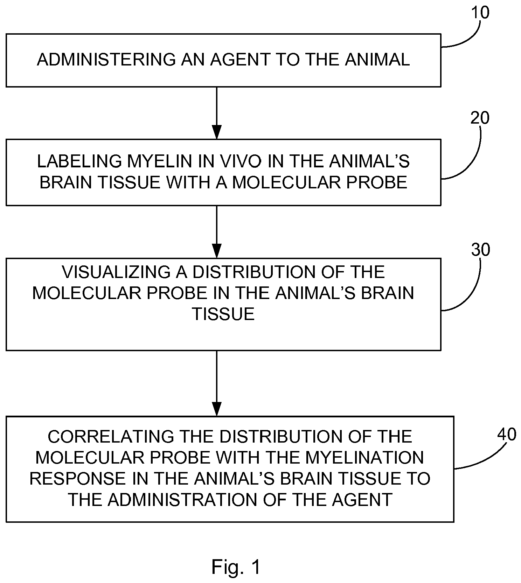

Fig. 1 illustrates a method of screening for a myelination response to an agent in an animal's brain in accordance with the present invention.

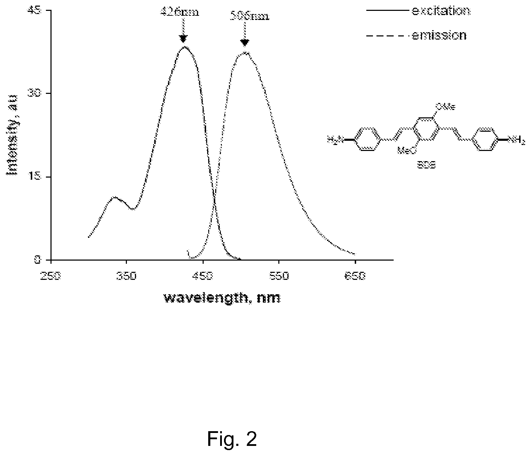

Fig. 2 illustrates excitation and emission spectra of (E,E)-1,4-bis(4-aminostyryl)-2-dimethoxy-benzene (BDB) (1 µM in DM50). Excitation spectra: emission at 510 nm (range 300-500 nm), bandwidth at 5 nm, scan at 120 nm/mm, integration time 0.5 sec. and maximal excitation wavelength at 426 nm. Emission spectra: excitation at 426 nm (range 430-650 nm), bandwidth at 5 nm, scan at 120 nm/min. integration time of 0.5 sec. and maximal emission wavelength at 506 nm.

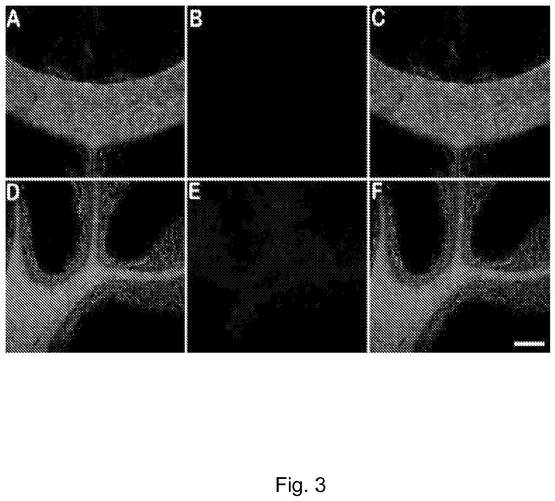

Fig. 3 illustrates photographs *in vitro* BDB staining of corpus callosum (green, coronal sections in A) and cerebellum (green, sagittal sections in D) in wild-type mouse brain compared with myelin basic protein (MBP) staining (red in B,E). Colocalization of corpus callosum and cerebellum with both staining are shown in C and F, respectively. Bar = 200 µm.

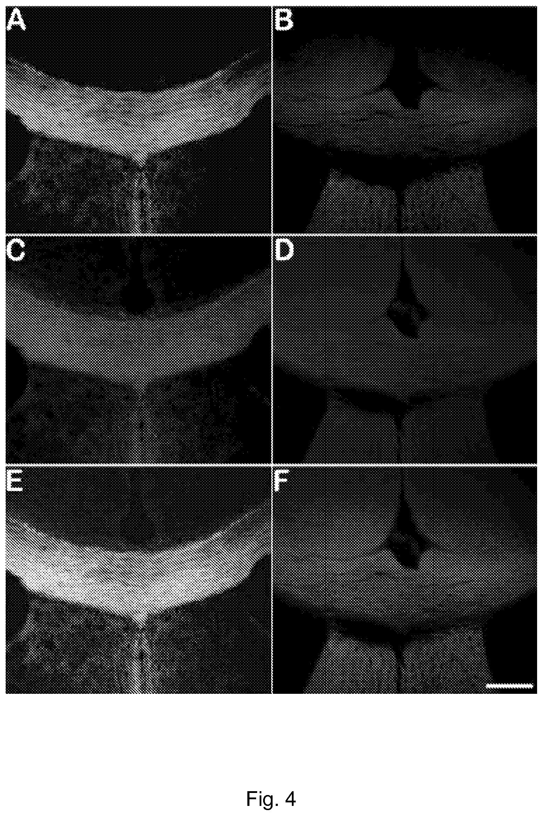

Fig. 4 illustrates photographs of in vitro BDB staining of corpus callosum in wild-type mouse brain (green in A) and quaking mouse brain (B) compared with MBP staining (red in CD). wt/wt, wild type; qk/qk, quaking mouse model. Colocalization of corpus callosum in both wt/wt and qk/qk are shown in E and F, respectively. Bar = 200 µm.

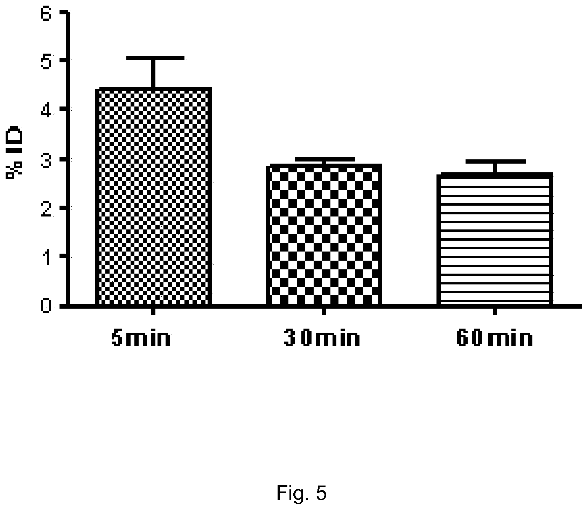

Fig. 5 illustrates a graph showing the level of BDB accumulation in mice brain. BDB (300 µl of 10 mM in 10% DMSO) was injected IV into normal control mice. At the indicated times after the injection, mice were sacrificed and brain levels of BDB determined by quantitative HPLC analysis as described in Materials and Methods. Data are from n=3 mice in each group and are shown as the mean ± SD of the whole brain concentration relative to the injected dose (%ID).

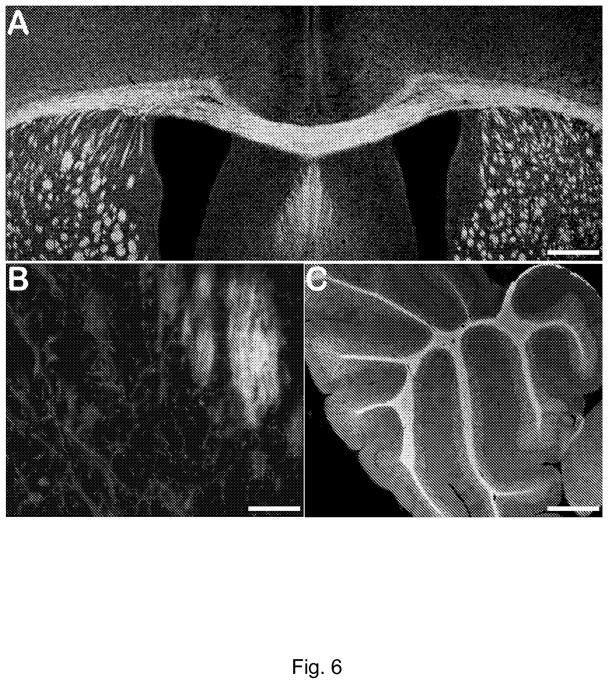

Fig. 6 illustrates photographs of ex vivo BDB staining of myelin sheaths in the corpus callosum (green in A) and cerebellum (green in C) colocalized with MBP staining (red) in the same sections. However, ex vivo staining of oligadendrocyte cell soma present in caudate putamen with BDB showed lack of staining in the cell bodies (B), whereas immunostaining for MBP was positive, indicating that BDB preferentially stains myelinated fibers. Bars: A,B = 500 µm; C = 50 µm.

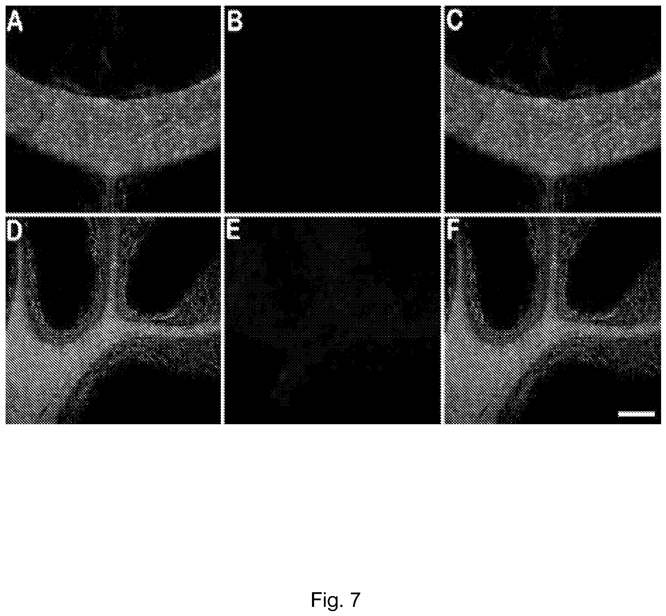

Fig. 7 illustrates photographs of staining of adjacent sections with a rat anti-mouse MBP primary antibody and a FITC-conjugated goat anti-rat IgG secondary antibody. No fluoromyelin was detected in either corpus callosum (B) or cerebellum (E). In contrast, abundant MBP-positive signals were visualized in the same regions at the adjacent brain section (A,D). Merged images of corpus callosum and cerebellum from fluoromyelin and MBP staining are shown in C and F, respectively. For injection, 0.5 ml of commercial fluoromyelin was used without any dilution. Bar = 200 µm.

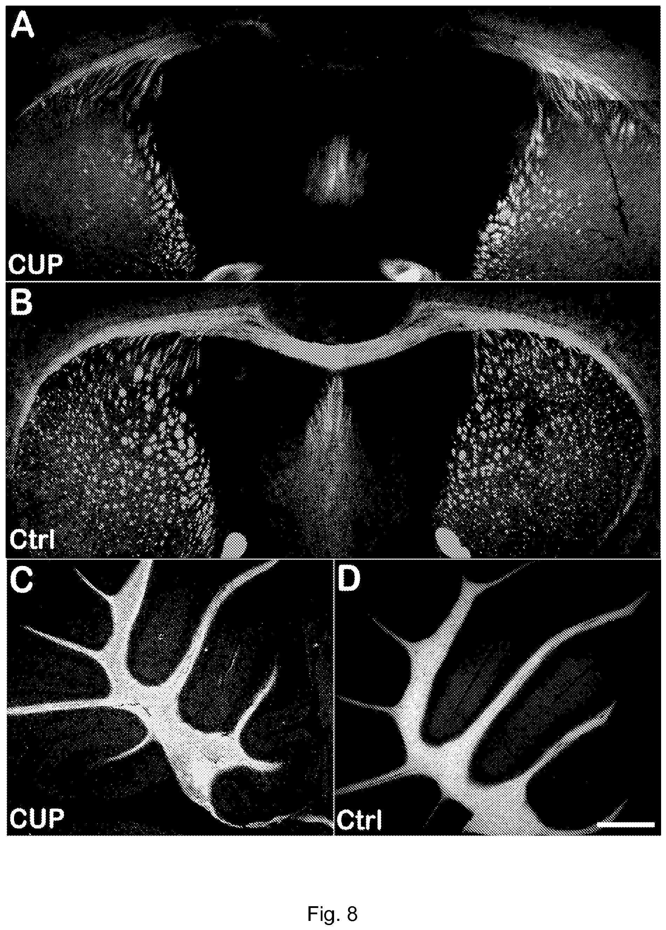

Fig. 8 illustrates ex vivo staining of corpus callosum in the cuprizone-treated mouse brain (A) and normal control mouse brain (B). Compared with the normally myelinated corpus callosum in the control mouse brain, significant demyelination was observed in the cuprizone-treated age- matched mouse littermate. Meanwhile, no significant demyelination was observed in cerebellum of cuprizone-treated mouse brain (C) compared with normal control mouse brain (D). CUP, cuprizone-treated; Ctrl, control. Bar 500 µm.

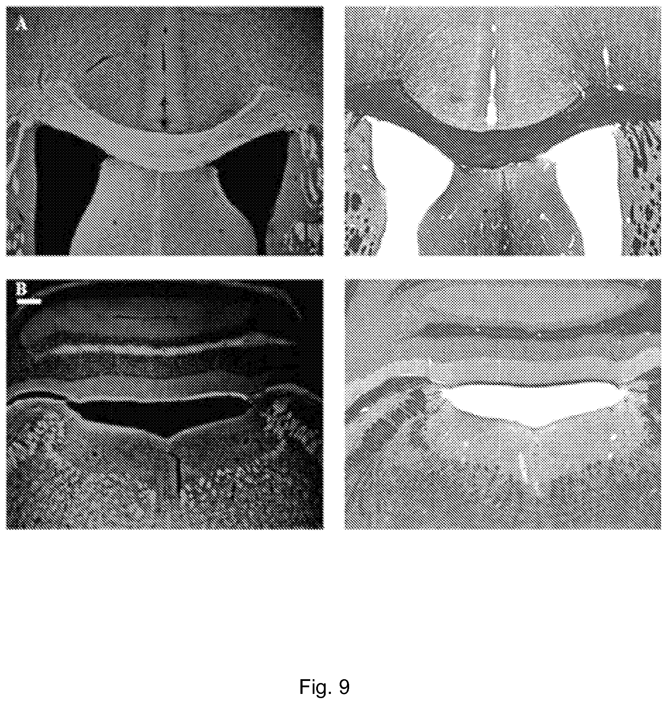

Fig. 9 illustrates photographs of *in vitro* CIC staining of intact myelinated white matter regions such as corpus callosum and cerebellum in wild type mouse brain compared with grey matter such as frontal cortex region.

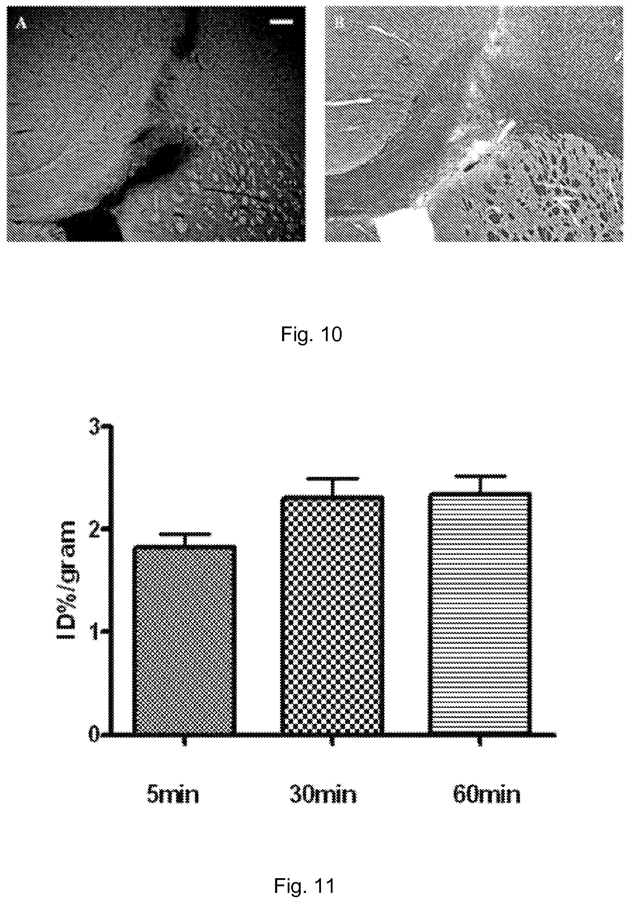

Fig. 10 illustrates photographs of the visualisation of demyelinated lesions using CIC. When CIC (40 mg/kg) was injected ip to mice at day 5 following treatment of lysolecthin, the demyelinated lesion in corpus callosum identified using black gold staining (A) were also visualized on adjacent sections with CIC (B). Both images were superimposed (C).
Fig. 11 illustrates a graph showing kinetic of CIC accumulation in mice brain. [¹¹C]CIC was injected i.v. into normal control mice. At the indicated times after the injection, the mice were sacrificed and brain were removed and weighed. Radioactivity in the brain was assayed in an automated gamma counter. The data were from 3 mice in each group, and are shown as the mean ± SD. of the whole brain concentration relative to the injected dose (%ID/g).

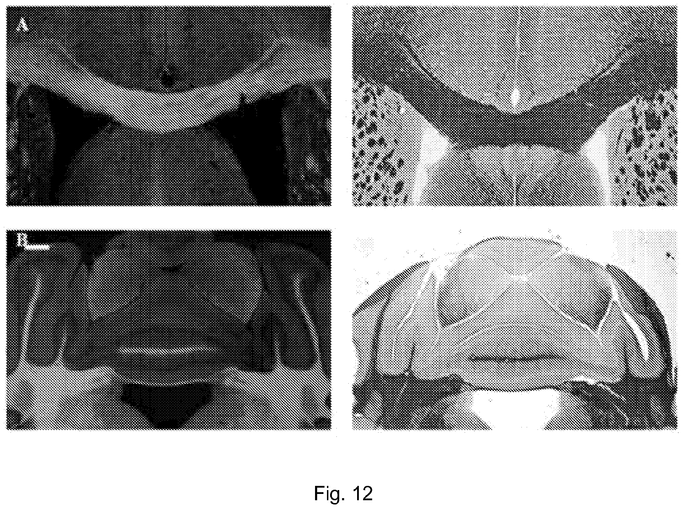

Fig. 12 illustrates photographs of *in situ* CIC staining of myelin sheaths in the corpus callosum (A, green) and cerebellum (B, green) colocalized with blackgold staining in the same sections. Bars in A and B = 500 µm, bar in B = 50 µm.

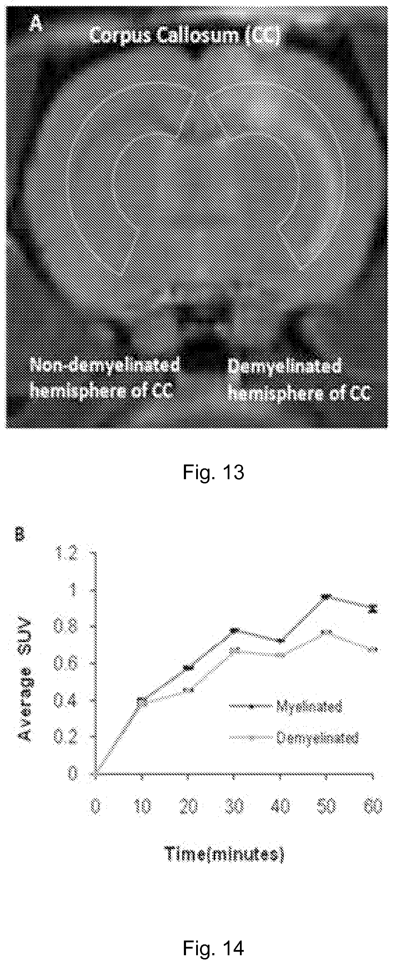

Fig. 13 illustrates a photograph of rat brains with focal demyelination. Demyelination was selectively induced in the left hemisphere of the corpus callosum (CC) following treatment with lysolecthin as shown by high-resolution MRI based on their hyperintensity. The symmetric regions in the right hemisphere without demyelination were used as control. Radioactivity concentrations were determined based on fusion of MRI and microPET images of corpus callosum.
Fig. 14 are plots showing the average radioactivity concentration in terms of standardized uptake value (SUV) as a function of time in demyelinated region (green) vs. intact myelinated region (blue) in corpus callosum of Fig. 13.

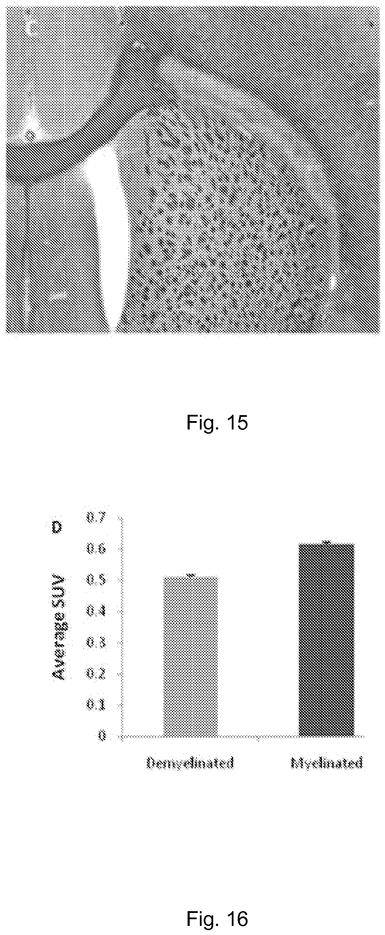

Fig. 15 is a graph showing the average SUV of demyelinated (green) and myelinated regions (blue) throughout the 60 min of scan with a p= 0.001 in corpus callosum of Fig. 13.
Fig. 16 illustrates a photograph showing that following *in vivo* imaging studies, the same demyelinated lesion in corpus callosum of Fig. 13 was confirmed by conventional blackgold staining.

### Detailed Description

The present invention relates to a molecular probe that upon administration to a mammal (e.g., systemic, parenteral, or intravenous administration) can readily enter the brain of the mammal and selectively localize to myelinated regions of the brain. The molecular probe binds to myelin membrane and not component of degenerating myelin fragments. The molecular probe can be readily visualized using conventional visualization techniques to indicate myelinated regions of the brain. The molecular probes can be used in a method of detecting a level of myelination *in vivo* in a subject, a method of detecting a myelin related disorder in a subject, a method of monitoring the remyelination effects of an agent in an animal, and a method of screening the myelination effects of an agent in an animal.

The molecular probe can include a fluorescent stilbenzene derivative that is less than about 700 daltons and has a relatively high binding affinity (Kd) of at least about 100nM to isolated myelin fractions but a relatively low binding affinity (Kd) of up to about 10µM to isolated non-myelin fractions. The fluorescent stilbenzene derivative can have an excitation spectra at a wavelength of about 300 nm to about 500 nm (emission at 506 nm) and emission spectra upon exciting at a wavelength of about 430 nm to about 650 nm (excitation at 426 nm).

According to the invention, the molecular probe is a fluorescent stilbenzene derivative having the following formula: 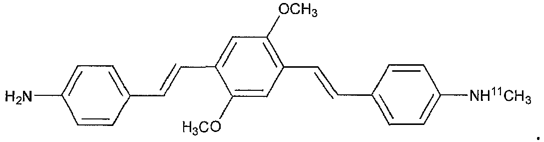 For exemplary reasons the following formula is disclosed: 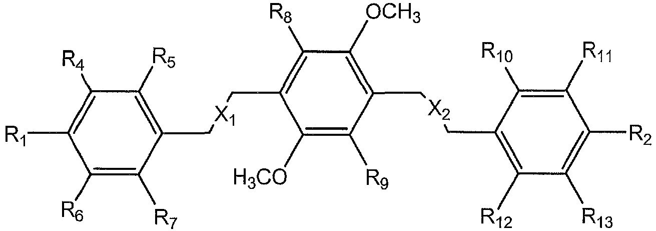 wherein R₁ and R₂ are each independently a hydrophilic or lipophilic group; wherein X₁ and X₂ are each independently a double or triple bond; and each R4-R13 is independently selected from the group consisting of H, F, Cl, Br, I, a lower alkyl group, (CH₂)ₙOR' (wherein n=1, 2, or 3), CF₃, CH₂-CH₂X, O-CH₂-CH₂X, CH₂-CH₂-CH₂X, O-CH₂-CH₂X (wherein X=F, Cl, Br, or I), CN, (C=O)-R', N(R')₂, NO₂, (C=O)N(R')₂, O(CO)R', OR', SR', COOR', R*ₚₕ,* CR'=CR'-R*ₚₕ*, CR₂'-CR₂'-R*ₚₕ* (wherein R*ₚₕ* represents an unsubstituted or substituted phenyl group, wherein R' is H or a lower alkyl group) or a salt thereof. In one aspect of the invention R₁ and/or R₂ can be selected from the group consisting of H, NO₂, NH₂, NHCH₃, N(CH₃)₂, OH, OCH₃, COOCH₃, SH, SCH₃, and alkyl derivatives thereof and each R₄-R₁₃ is H.

The molecular probe can include a fluorescent stilbenzene derivative which has been shown for exemplary reasons having the following formula: 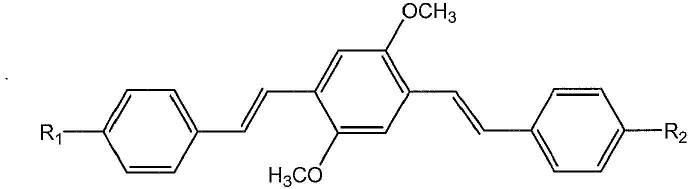 wherein R₁ and R₂ are each independently selected from the group consisting of H, NO₂, NH₂, NHCH₃, N(CH₃)₂, OH, OCH₃, COOCH₃, SH, SCH₃, and alkyl derivatives thereof or a salt thereof.

The molecular probe can include a fluorescent stilbenzene derivative which has been shown for exemplary reasons having the following formula: 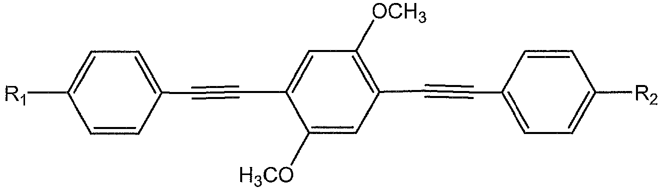 wherein R₁ and R₂ are each independently selected from the group consisting of H, NO₂, NH₂, NHCH₃, N(CH₃)₂, OH, OCH₃, COOCH₃, SH, SCH₃, and alkyl derivatives thereof or a salt thereof.

In a further example, the molecular probe can be a (E,E)-1,4-bis(4'-aminostyryl)-2-dimethoxy-benzene (BDB) which has the following general structure: 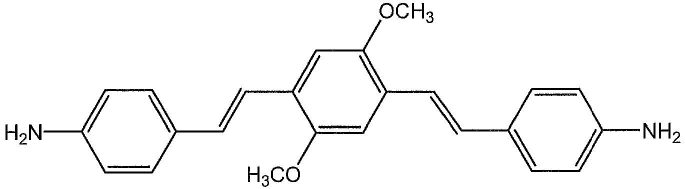 or a salt thereof.

The foregoing formulae represent the general structures of fluorescent stilbenzene compounds found to be effective molecular probes for labeling myelin in vivo as well as in vitro as described in the examples below. They are characterized by their ability to enter the brain and selectively localize in the myelinated regions via direct binding to myelin membranes and not bind to degenerating myelin fragments.

When referring to the terms "fluorescent stilbenzene compound" or "compound" in the specification and the claims, it is intended that the terms encompass not only the specified molecular entity but also its pharmaceutically acceptable, pharmacologically active analogs, including, but not limited to, salts, esters, amides, prodrugs, conjugates, active metabolites, and other such derivatives, analogs, and related compounds.

By "pharmaceutically acceptable" is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" derivative or analog, refers to a derivative or analog having the same type of pharmacological activity as the parent compound and approximately equivalent in degree.

As used herein, the term "pharmaceutically acceptable salts" or complexes refers to salts or complexes that retain the desired biological activity of the parent compound and exhibit minimal, if any, undesired toxicological effects. Nonlimiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, palmoic acid, alginic acid, polyglutamic acid, naphthalenesulfonic acids, naphthalenedisulfonic acids, and polygalacturonic acid; (b) base addition salts formed with cations such as sodium, potassium, zinc, calcium, bismuth, barium, magnesium, aluminum, copper, cobalt, nickel, cadmium, sodium, potassium, and the like, or with an organic cation formed from N,N-dibenzylethylene-diamine, ammonium, or ethylenediamine; or (c) combinations of (a) and (b); e.g., a zinc tannate salt or the like.

Typically, the molecular probe can be formulated into solution prior to use. In one example, a molecular probe solution includes a 10mM molecular probe solution. A molecular probe solution can also contain saline, DMSO, and HCL. One skilled in the art can utilize the molecular probe with pharmaceutical carriers and/or excipients in varying concentrations and formulations depending on the desired use.

The molecular probe can be radiolabeled to aid in the detection of the molecular probe once it binds to myelin. A 'radiolabel' as used herein is any compound that has been joined with a radioactive substance. Examples of radiolabels include positron emitting 11C and 18F radiolabels.

The molecular probe can be coupled to a chelating group (with or without a chelated metal group) to improve the MRI contrast properties of the molecular probe. In one example, as disclosed in U.S. Patent No. 7,351,401, the chelating group can be of the form W-L or V-W-L, wherein V is selected from the group consisting of -COO-, -CO-, -CH2O- and -CH2NH-; W is -(CH2)n where n=0,1,2,3,4, or 5; and L is: 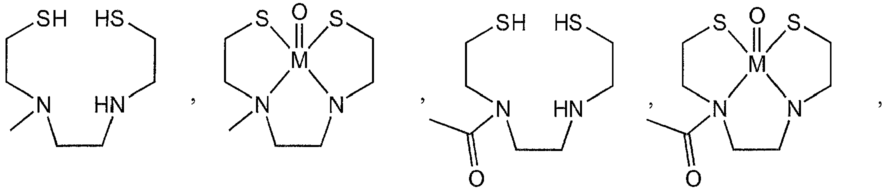 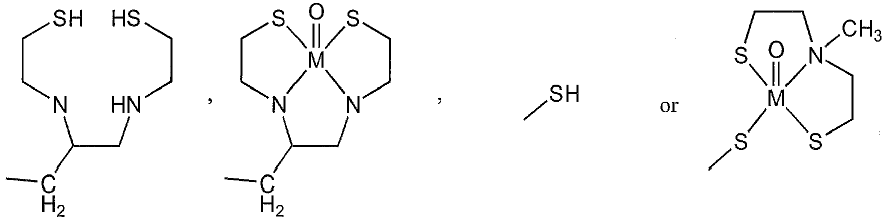 wherein M is selected from the group consisting of Tc and Re; or 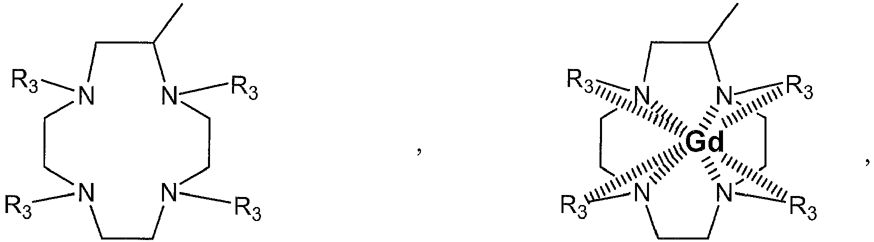 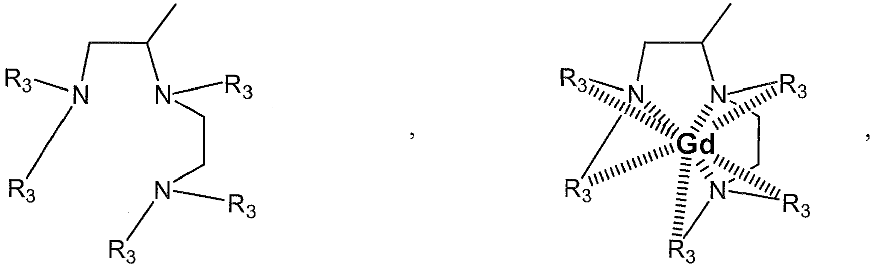 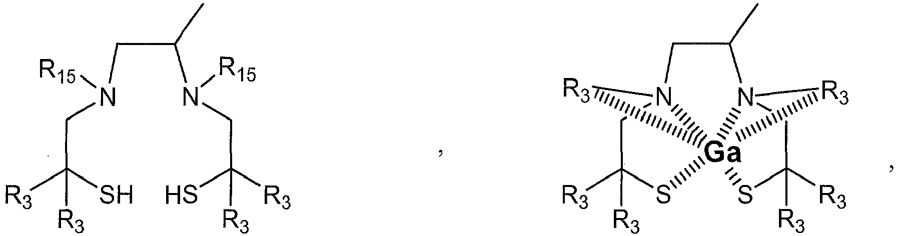 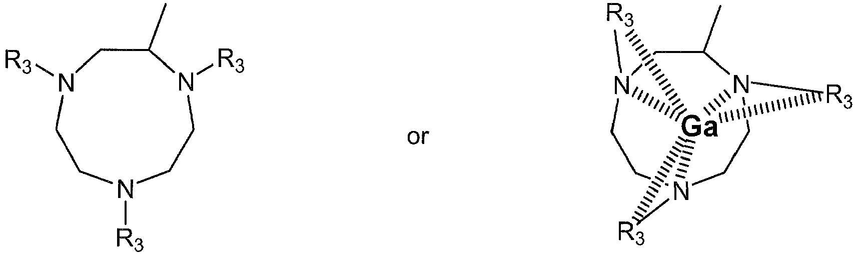 wherein each R3 is independently is selected from one of: H, 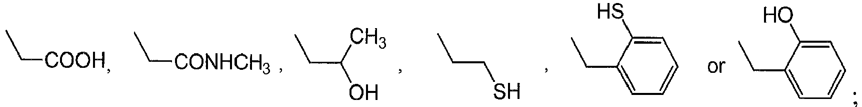 or a myelin binding, chelating compound (with or without a chelated metal group) or a water soluble, non-toxic salt thereof of the form: 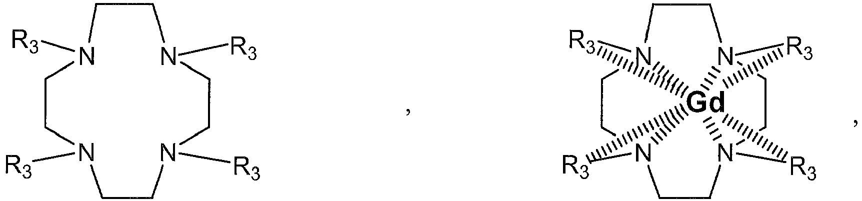 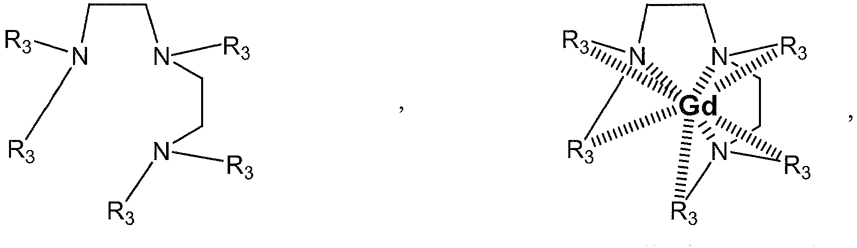 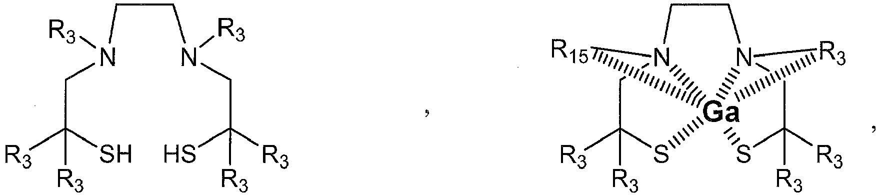 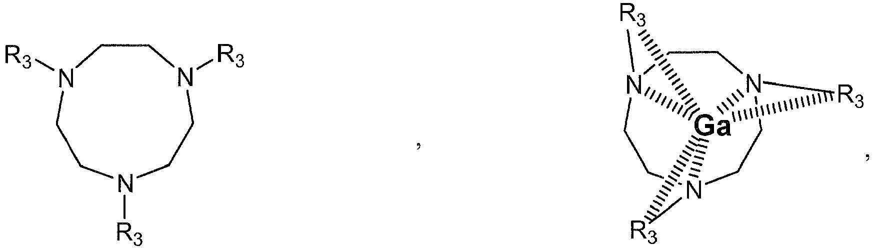 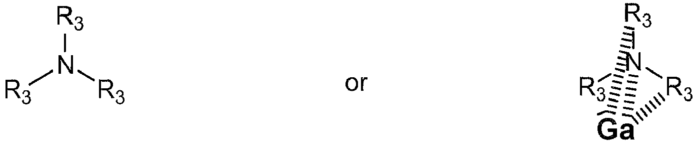 wherein each R₃ independently is selected from one of: H,

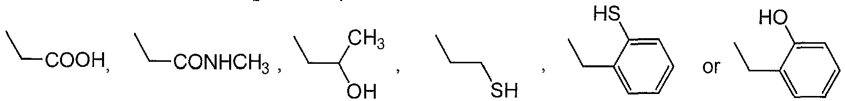

The chelating group can be coupled to the central benzene group, at least one terminal benzene groups, or the R1 or R2 groups. In one example, the chelating group can be coupled to terminal amino R1 and/or R2 group through carbon chain link. The carbon chain link can comprise, for example about 2 to about 10 methylene groups and have a formula of, for example, (CH2)n, wherein n = 2 to 10.

In one example, the molecular probe with the chelating group can have the following formula: 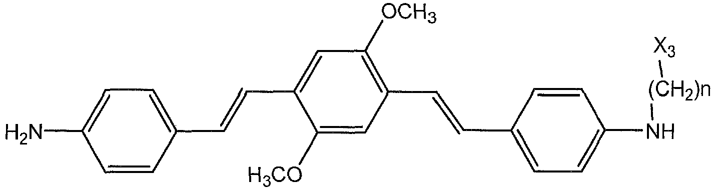 wherein X₃ is a chelating group and n is 2 to 10; or a salt thereof.

In another example, the molecular probe with the chelating group can have the following formula: 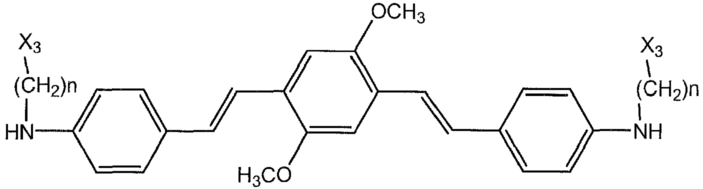 wherein X₃ is a chelating group and n is 2 to 10; or a salt thereof.

In a further example, the molecular probe with the chelating group can have the following formula: 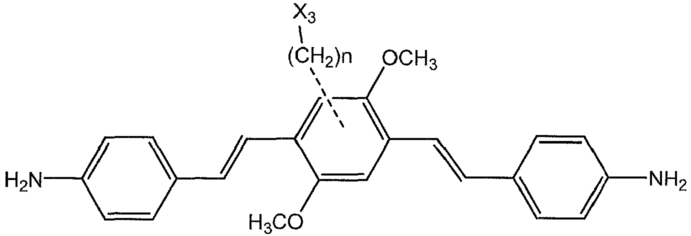 wherein X₃ is a chelating group and n is 2 to 10; or a salt thereof.

The molecular probe can be coupled to a near infrared group to improve the near infrared imaging of the molecular probe. Examples of near infrared imaging groups that can be coupled to the molecular probe include: 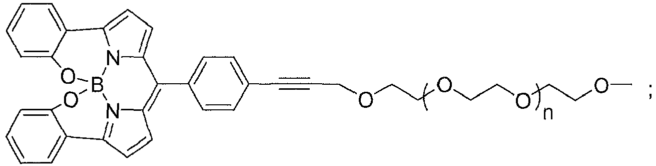 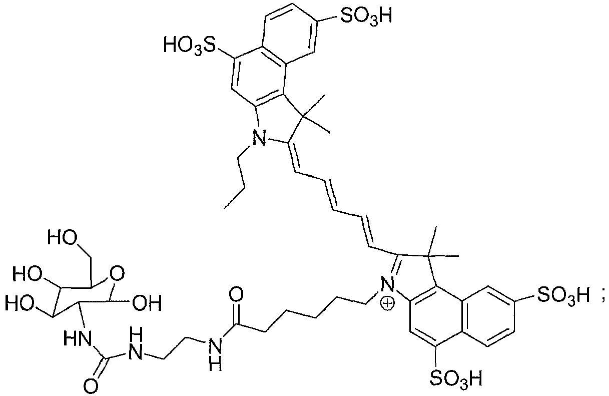 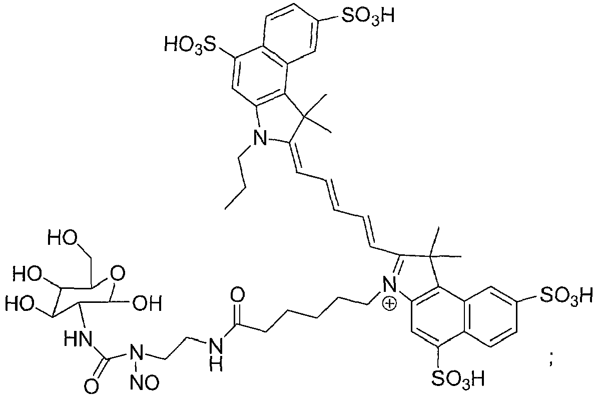 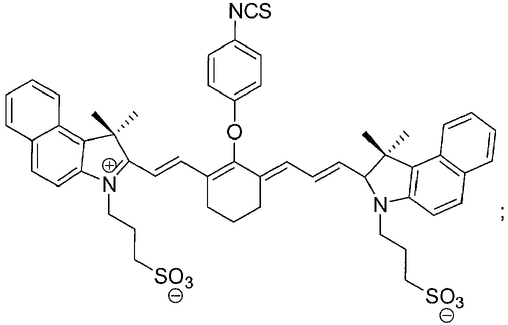 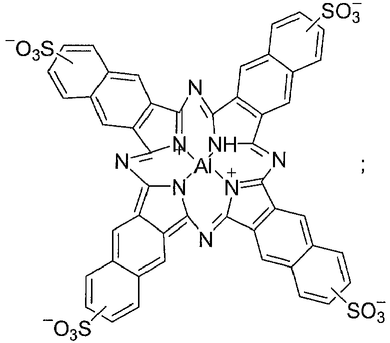 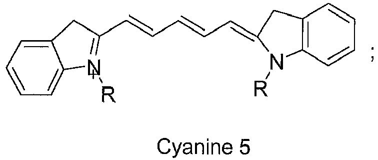 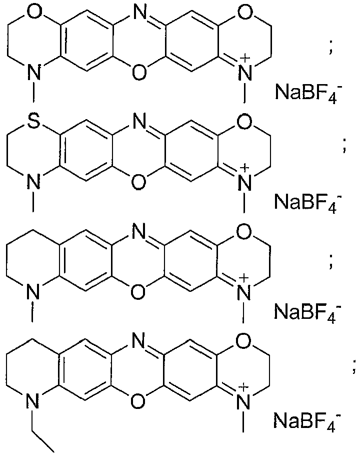 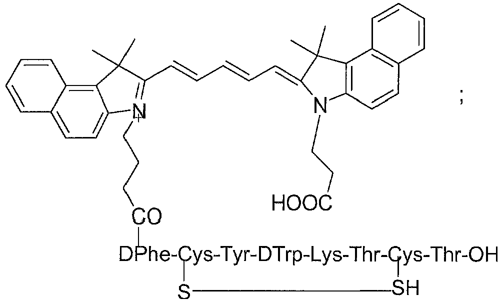 and

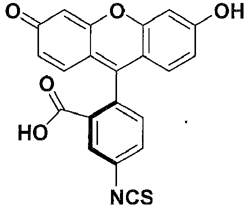

These near infrared imaging groups are disclosed in, for example, Tetrahedron Letters 49(2008) 3395-3399; Angew. Chem. Int. Ed. 2007, 46, 8998-9001; Anal. Chem. 2000, 72, 5907; Nature Biotechnology vol 23, 577-583; Eur Radiol(2003) 13: 195-208; and Cancer 67: 1991 2529-2537.

The near infrared imaging group can be coupled to the central benzene group, at least one terminal benzene groups, or the R1 or R2 groups. In one example, the near infrared imaging group can be coupled to at least one terminal benzene group.

In one example, the molecular probe with the near infrared imaging group can have the following formula: 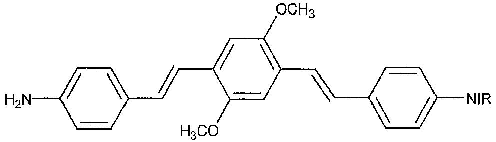 wherein NIR is a near infrared imaging group; or a salt therof.

By way of example, the molecular probe can include a compounds having the following formula: 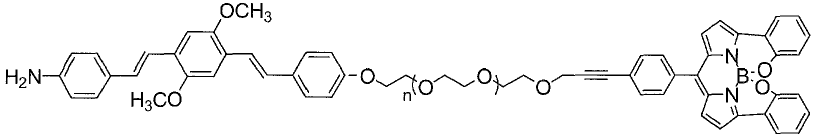 wherein n is 3 to 10; or a salt thereof.

In another example, the molecular probe with the chelating group can have the following formula: 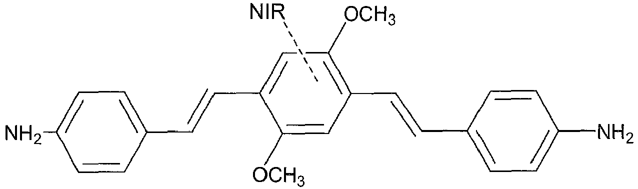 wherein NIR is a near infrared imaging group; or a salt thereof.

The molecular probes described herein can be contacted with an animal's brain tissue and utilized for labeling and detecting myelinated regions of an animal's brain tissue. Myelinated regions of an animal's brain are typically found in the white matter of the brain in the myelin sheaths of neuronal axons. Myelin is an outgrowth of glial cells, more specifically oligodendrocytes, which serve as an electrically insulating phospholipid layer surrounding axons of many neurons. For purposes of the present invention, an animal's brain tissue is typically a mammal's brain tissue, such as a primate, e.g., chimpanzee or human; cow; dog; cat; a rodent, e.g., guinea pig, rat, mouse; rabbit; or a bird; reptile; or fish.

The molecular probes described herein can be used for the in vivo detection and localization of myelinated regions of an animal's brain. The molecular probe can be administered to the animal as per the examples contained herein, but typically through intravenous injection. "Administered", as used herein, means provision or delivery molecular probes in an amount(s) and for a period of time(s) effective to label myelin in an animal's brain tissue. The molecular probes can be administered to the animal can be enterally or parenterally in a solid or liquid. Enteral route includes oral, rectal, topical, buccal, and vaginal administration. Parenteral route includes intravenous, intramuscular, intraperitoneal, intrastemal, and subcutaneous injection or infusion.

An example of a dosing regimen is to administer about 40- about 50 mg/kg by weight to the animal. In one example at 5 min, the brain concentration of probe can range between about 4 % to 24 % ID/g to ensure sufficient visualization of the myelinated regions of the brain.

The molecular probes of the present invention can be used for neuroanatomical or neuropathological studies. Researchers studying normal brains can employ this method to examine the morphology and distribution of myelinated tissue in an animal. "Distribution" as used herein is the spatial property of being scattered about over an area or volume. In this case the "distribution of myelinated tissue" is the spatial property of myelin being scattered about over an area or volume included in the animal's brain tissue. Researchers interested in neurotoxicology and neuropathology can also use this method in several ways. One way is to infer demyelination by the absence of the molecular probe labeling compared to normal control brains. A second way is to study morphological changes in the myelin such as a fragmented or beaded appearance of the myelin sheath. In yet another embodiment of the present invention, one skilled in the art can assess and quantify changes in myelin content in vivo.

In other aspects of the present invention, myelin in an animal's brain tissue can be visualized and quantified using an in vivo imaging modality. The molecular probe may be visualized any time post administration depending on the application as typical molecular probes embodied in the present invention have a low clearance rate due to specific binding in the myelinated regions (e.g. at 60 min, the brain concentration of probe can be ≤50% of 5 min value to ensure that half time retention in normally myelinated brain is 60 min or longer).

An in vivo imaging modality as used herein is an imaging modality capable of visualizing molecular probes described herein in vivo (within a living organism). An example of an in vivo imaging modality is positron emission tomography (PET). PET is a functional imaging technique that can detect chemical and metabolic change at the molecular level. To function as a PET imaging molecular probe, embodiments of the present invention must meet a set of biological requirements known to the skilled artisan, some of which may include lipophilicity, binding affinity, binding specificity, brain uptake, retention, and metabolism. Another example of an in vivo imaging modality is MicroPET. MicroPET is a high resolution positron emission tomography scanner designed for imaging small laboratory animals. Other examples of imaging modalities that can be used in accordance with the present invention include magnetic resonance imaging (MRI), near infrared (NIR) imaging, fluorescent microscopy, and mutiphoton microscopy.

For directly monitoring myelin changes in the white matter of a subject, embodiments of the invention can readily penetrate the blood-brain barrier (BBB) and directly bind to the myelinated white matter in proportion to the extent of myelination. Radiolabeled molecular probes of the present invention can be used in conjunction with PET as imaging markers to directly assess the extent of total lesion volumes associated with demyelination. This can provide a direct clinical efficacy endpoint measure of myelin changes and identify effective therapies aimed at protection and repair of axonal damages.

The molecular probes of the present invention can also be used to diagnose a myelination related disorder in an animal through the use of in vivo myelin labeling. Thus, in certain embodiments of the present invention, solutions containing the molecular probes describe herein can be used in the detection of myelin related disorders in an animal.

Methods of detecting a myelin related disorder include the steps of labeling myelin in vivo in the animal's brain tissue with a molecular probe described herein, visualizing a distribution of the molecular probe in the animal's brain tissue as described above and in the examples, and then correlating the distribution of the molecular probe with a myelin related disorder in the animal. In one example of detecting a myelin related disorder, the methods described herein can be used to compare myelinated axonal regions of the brain in the normal tissues of control populations to those of a suspect animal. If the suspect animal has a myelin related disorder, myelin may be virtually absent in lesioned areas thus indicating the presence of a myelin related disorder.

Myelination disorders can include any disease, condition (e.g., those occurring from traumatic spinal cord injury and cerebral infarction), or disorder related to demylination, remylination, or dysmyelination in a subject. A myelin related disorder as used herein can arise from a myelination related disorder or demyelination resulting from a variety of neurotoxic insults. Demyelination is the act of demyelinating, or the loss of the myelin sheath insulating the nerves, and is the hallmark of some neurodegenerative autoimmune diseases, including multiple sclerosis, transverse myelitis, chronic inflammatory demyelinating polyneuropathy, and Guillain-Barre Syndrome. Leukodystrophies are caused by inherited enzyme deficiencies, which cause abnormal formation, destruction, and/or abnormal turnover of myelin sheaths within the CNS white matter. Both acquired and inherited myelin disorders share a poor prognosis leading to major disability. Thus, some embodiments of the present invention can include methods for the detection of neurodegenerative autoimmune diseases in an animal and more specifically the detection of multiple sclerosis in an animal.

Another embodiment of the present invention includes a method of monitoring the efficacy of a remyelination therapy in an animal. Remyelination is the repair of damaged or replacement of absent myelin in an animal's brain tissue. The methods described include the steps of labeling myelin in vivo in the animal's brain tissue with a molecular probe described herein, then visualizing a distribution of the molecular probe in the animal's brain tissue (e.g. with a in vivo imaging modality as described herein), and then correlating the distribution of the molecular probe as visualized in the animal's brain with the efficacy of the remyelination therapy. It is contemplated that the labeling step can occur before, during, and after the course of a therapeutic regimen in order to determine the efficacy of the therapeutic regimen. One way to assess the efficacy of a remyelination therapy is to compare the distribution of the molecular probe before remyelination therapy with the distribution of the molecular probe after remyelination therapy has commenced or concluded.

Remyelination therapy as used herein refers to any therapy leading to a reduction in severity and/or frequency of symptoms, elimination of symptoms and/or underlying cause, prevention of the occurrence of symptoms and/or their underlying cause, and improvement or remediation of damage related to demyelination. For example, a remyelination therapy can include administration of a therapeutic agent, therapies for the promotion of endogenous myelin repair, or a cell based therapy (e.g., a stem-cell based therapy).

In another embodiment of the present invention, methods are provided for screening for a myelination response in an animal's brain tissue to an agent. As shown in Fig. 1, at 10, the method includes the initial step of administering an agent to the animal. At 20, myelin in the animal's brain tissue is labeled in vivo with a molecular probe in accordance with the present invention. At 30, a distribution of the molecular probe in the animal's brain tissue is then visualized using a conventional visualization modality. Finally, at 40, the distribution of the molecular probe with the myelination response in the animal's brain tissue is correlated to the agent. One way to assess the myelination response in the animal's brain tissue is to compare the distribution of the molecular probe in an animal's brain tissue, which has been treated with a suspect agent with the distribution of the molecular probe in the brain tissue of a control population. "Control Population" as used herein is defined as a population or a tissue sample not exposed to the agent under study but otherwise as close in all characteristics to the exposed group as possible.

The molecular probes described herein can be used to determine if an agent of interest has the potential to modulate demyelination, remyelination, or dysmyelination of axonal regions of an experimental animal's brain tissue.

### EXAMPLES

### EXAMPLE 1

We developed a myelin-specific probe that readily enters the brain and selectively bind to myelin sheaths. The probe includes (E,E)-1, 4-bis(4'-aminostyryl)-2-dimethoxy-benzene (BDB). BDB is a fluorescent stilbenzene derivative that is selectively retained in white matter by binding to myelin. In the absence of myelin sheaths, as occurs in the quaking mouse brain, BDB binding was virtually undetectable. BDB selectively stains intact myelin sheaths in normal mice in situ following IV injection. BDB brain uptake also allows visualization of demyelinated lesions in cuprizone-treated mice, yielding images similar to those observed in histochemical staining using antibody or other myelin dye-staining procedures.

### Chemical Synthesis and Characterization of BDB

Detailed synthetic procedures of BDB is shown below. The chemical structure of BDB was confirmed by proton nuclear magnetic resonance spectroscopy and high-resolution mass spectrometry.

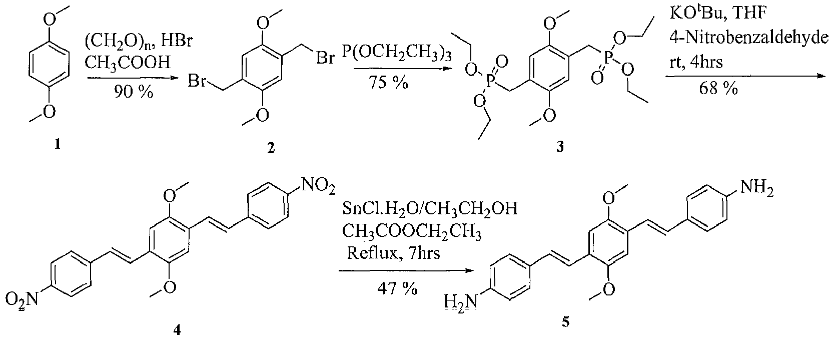

### Animal Preparation and Cuprizone Treatment

This study used wild-type (n=3), quaking mutant (qk^{v}) (n=3), and cuprizone-treated (n=6) mice. Homozygous qk^{v} mutant mice and normal female C57BL/6 mice (6 to 8 weeks of age) were obtained from Jackson Laboratory (Bar Harbor, ME). The cuprizone mouse model of demyeination was induced by feeding 6- to 8-week-old female C57BL/6 mice a diet of milled mouse chow containing 0.2% of the copper chelator, cuprizone (Sigma-Aldrich; St Louis, MO) for 6 weeks. At this dose, demyelination is largely restricted to the corpus callosum, although there is a dramatic reduction of myelin protein gene expression throughout the CNS. Maximum demyelination is normally seen following about 6 weeks of treatment resulting from a virtual complete loss of oligodendrocytes from the corpus callosum within 2 to 3 weeks. Brains were harvested from these mice at the peak of demyelination (i.e., 6 weeks post treatment).

### In Vitro Staining

Two-month-old C57BL/6 mice were deeply anesthetized and perfused transcardially with 4% parafonnaldehyde in PBS (pH 7.3). Brains were then removed from the calvarium, immersion postfixed in the same fixative solution, dehydrated in 20% sucrose, embedded in freezing compound (OCT; Miles Scientific), cryostat sectioned at 10 µm, and mounted on superfrost slides (Fisher Scientific). Sections were then incubated in a solution of BDB (10 µM) in 1% DMSO/PBS for 20 mm at room temperature. Excess BDB was removed by briefly rinsing the sections in PBS before coverslipping with fluoromount-G mounting media (Vector Laboratories; Burlingame, CA). Sections were then examined with Olympus IX 51 microscope equipped with an Axiocam MRm digital camera and Axiovision 4.3 software (Olympus; Tokyo, Japan). In some cases, tissue sections were double stained with anti-myelin basic protein (MBP) monoclonal antibody (MAb) (see below).

For IHC, sections mounted on slides were incubated in a solution containing anti-MBP MAb primary antibody (rabbit anti-MBP, 1:300; Zymed Laboratories, South San Francisco, CA) diluted in 1% normal donkey serum overnight at 4C. Following three rinses with PBS, sections were incubated in donkey anti-rabbit Rhodamine Red-X-conjugated secondary antibody or goat anti-rat IgG fluorescein-conjugated secondary antibody (Jackson ImmunoResearch Laboratories; West Grove, PA) (diluted 1:200 in PBS with 1% normal donkey serum) for 1 hr at 37C, then washed three times for 5 min each with PBS. DAPI (400 ng/ml in PBS) staining was performed to visualize nuclei following washes with PBS. Images were obtained on an Olympus IX 51 microscope equipped with an Axiocam MRm digital camera and Axiovision 4.3 software.

### Quantitation of BDB Brain Uptake

Two-month-old Swiss Webster mice weighing 20-25 g were injected with a 0.4-ml solution containing saline (85%), DMSO (10%), MCI (5%, 0.3 nM), and 1.0 mg of BDB in the tail vein (n=3 mice per group). At 5, 30, and 60 minutes post-injection, mice were sacrificed by heart puncture, and brains were rapidly removed, weighed, and homogenized together with an internal standard (BBD). After extraction, solvent was evaporated and the residue was redissolved in ethyl acetate. The concentration of parent BDB was determined by HPLC using a Phenomenex analytical column (Luna C18, 5 µm, 250 X 4.60 mm; Phenomenex, Torrance, CA), acetonitrile: 3,3-dimethylglutaric acid (DMGA), pH 7, diluted 70:30, and corrected by internal standard.

### Ex Vivo Characterization of BDB

In this experiment, 10-100 mg/kg of BDB was injected IV through the tail vein of control and cuprizone-treated mice (6 weeks). Animals were sacrificed 18 hr after injection and processed as described above. An optimal concentration (40-50 mg/kg) of injected BDB was determined in control animals and the same concentration was used in cuprizone-treated mice for further ex vivo studies. Similarly, 0.5 ml of commercially available fluoromyelin (Molecular Probes; Eugene, OR) was injected without further dilution to examine the entry of BDB into the brain and ex vivo staining of myelin sheaths in the brain.

### Physiochemical Properties of BDB

BDB is a fluorescent compound and is soluble in CH₂Cl₂, DMSO, and in most other organic solvents. Excitation and emission spectra of BDB (1 µM in DMSO) recorded using a Cary Eclipse fluorescent spectrophotometer (Variant Inc.; Palo Alto, CA) are shown in Fig. 2. Maximal excitation and emission peaks were found at 426 nm and 506 nm, respectively.

### BDB Stains Intact Myelin Sheaths In Vitro

Myelin-binding properties of BDB were first examined by in vitro staining of frozen brain sections from wild-type mice. For comparison, immunohistochemical (IHC) staining for myelin-specific MBP was also conducted in adjacent sections. Both corpus callosum and cerebellar white matter were then examined by fluorescent microscopy. At 10 µM concentration, BDB selectively labeled intact myelin sheaths in both corpus callosum (Fig. 3A) and cerebellar white matter (Fig. 3D). The pattern of myelin sheath staining detected by BDB was virtually identical to the pattern detected by MBP staining (Fig. 3B and 3E). Overlap between BDB and MEP staining is shown in the merge image (Fig. 3C and 3F, respectively). These observations indicated that BDB was a specific marker for myelin sheaths in the corpus callosum and cerebellum.

Specificity of BDB for myelin was tested by comparing staining in myelin-deficient quaking mice compared with age-matched control littermates. The quaking mouse is a mutant model of dysmyelination, resulting in complete CNS demyelination shown by a lack of myelin staining of the corpus callosum with BDB (Fig. 4B) and MBP (Fig. 4D) compared with intense labeling in age-matched littermate controls (BDB, Fig. 4A and MBP, Fig. 4C). Colocalization of BDB and MBP staining of corpus callosum in wild-type and quaking mice are shown in Figs. 4E and 4F, respectively.

### BDB Permeability in Mouse Brain

Brain permeability of BDB was evaluated in normal mice using HPLC analysis. Mice were given a single IV injection of BDB solution (0.3 ml, 10 mM), sacrificed after 5, 30, and 60 min, and brain concentrations of BDB determined. As shown in Fig. 5, brain uptake already reached 4.43 ± 1.10% of the injected dose (ID) within 5 min post injection. At 30 min, brain concentrations decreased slightly (to 2.99 ± 0.28% ID) but did not show any further decrease when measured after 60 mm (2.70 ± 0.33% ID).

### BDB Stains Myelin in Living Mice

Following in vitro studies, we investigated the ability of BDB to monitor myelin contents ex vivo in the mouse brain. A dose of 1.0 mg BDB (50 mg/kg) was injected via the tail vein into wild-type mice. Eighteen hr post injection, mice were perfused (see above) and brains were removed and sectioned as described above. BDB staining of myelin was then directly examined under fluorescent microscopy. As shown in Fig. 6, BDB entered the brain and selectively labeled myelin sheaths of the corpus callosum (Fig. 6A) and cerebellum (Fig. 6C) of the wild-type mice. Subsequent immunostainirig for MBP revealed that BDB bound more selectively to myelin fibers, because MBP stained oligodendrocyte (OL) cell soma and processes in the caudate putamen (CPu) (Fig. 6B) under the same conditions. In cerebellum, BDB staining was confined to white matter tracts, whereas MBP stained fibers in the granule cell layer, suggesting that BDB binds preferentially to compacted myelin.

Following ex vivo studies with BDB, a similar study was carried out with fluoromyelin for comparison. Thus, we injected 0.5 ml of commercial fluoromyelin in an undisclosed original concentration, which was as high as 300 times that used for in vitro staining. Three hr postinjection, the mouse brain was treated under the above conditions and sectioned. No fluoromyelinwas detected in various myelinated regions such as corpus callosum (Fig. 7B) and cerebellum (Fig. 7E). As a result, myelinated structures could not be stained ex vivo by fluoromyelin, although the presence of myelin sheaths in both regions were confirmed by MBP immunostaining in adjacent sections (Fig. 7A and 7B). This indicated that fluoromyelin is not permeable across the BDB. Thus, BDB offers a major advantage in its potential to stain myelin in vivo.

### BDB Detects Demyelinated Lesions in Living Cuprizone-treated Mice

Wild-type C57BL/6 mice were treated with the selective neurotoxin cuprizone for 6 weeks to induce demyelination, after which we injected BOB into mice as described above and prepared brain sections 18 hr later. Under these conditions, cuprizone is known to induce significant demyelination in the corpus callosum (Matsushima and Morell 2001). As shown in Fig. 8A, BDB readily entered the brain and selectively detected the chemically induced demyelinated lesions found in the corpus callosum in comparison to the normal control brain (Fig. 8B). As expected, little or no differences in BDB fluorescence were observed in cerebellum (Fig. 8C and 8D).

Based on the above in vitro and ex vivo studies, we have demonstrated that the fluorescent probe BDB can be used as a specific histochemical stain for myelin. This is based on the following observations: (1) BDB selectively stained intact myelin sheaths present in the corpus callosum of the wild-type mouse brain and not components of degenerating myelin fragmenssts. (2) BDB staining was not observed in the corpus callosum in the myelin-deficient quaking mutant mice. (3) BDB readily penetrated the BBB and accumulated in the brain following IV injection. (4) BDB readily allowed detection of demyelinated lesions found in the corpus callosum of cuprizone-treated mice in situ following IV injection but not in the cerebellum where cuprizone does not induce lesions.

BDB binds only to intact myelin sheaths not components of degenerating myelin fragments. The requirement for intact myelin is also evident in the staining patterns observed (see Figs. 3F and 6C), where one can see IHC staining for MBP outside of the white matter tracts to which BDB staining is restricted. Similarly, in frontal cortex deficient in myelin sheaths, no BDB staining was observed, whereas IHC staining for MBP was still positive due to staining of free MBP localized in the OL cytoplasm in the absence of myelin sheaths (Fig. 6B). Together these findings are consistent with BDB binding to a structural feature present only in compact myelin and not to either individual myelin protein or to a lipid component.

Development and availability of a BBB-permeable fluorescent probe BDB complements conventional histochemical techniques. Existing myelin stains such as fluoromyelin do not penetrate the BBB and thus are limited to in vitro histopathological studies (Fig. 7). BDB, therefore, provides a means to carry out myelin detection in vivo.

### EXAMPLE 2

The following example discloses a novel myelin-imaging agent, termed CIC that has been used for microPET studies in lysolecthin-treated rat model of focal demyelination.

### Chemical Synthesis of CIC

Reagents and solvents were purchased from Sigma-Aldrich and used without further purification unless otherwise stated. Intermediates were purified by column chromatography on silica gel G60 (230-400 mesh) using reagent grade solvents. ¹H- and ¹³C NMR spectra were recorded on a Bruker AMX 400 MHz spectrometer. Chemical shifts were reported in part per million (ppm, δ) downfield from tetramethylsilane. Proton coupling patterns were described as a singlet (s), doublet (d), triplet (t), quartet (q), multiplet (m), and broad (br). Analytical TLC was performed on silica gel F-254 aluminum plates, with visualization under UV (254 nm).

### 1,4-Bis(bromomethyl)-2,5-dimethoxylbenzene

A 33 % solution of HBr in glacial acetic acid (14 mL, 0.08 mol) was added dropwise to a suspension of 1,4-dimethoxybenzene (5.0 g, 0.04 mol) and paraformaldehyde (2.37 g, 0.08 mol) in glacial acetic acid (25 mL). The mixture was stirred for one hour at 50 C. The doubly bromomethylated product precipitated and was isolated by filtration, dissolved in chloroform (80 mL) and the solution dried with anhydrous sodium sulfate. After filtration, partial evaporated of the solvent under vacuum enabled precipitation of colorless crystals (10.14 g, 90 %). ¹H NMR (400 MHz, CDCl₃); δ 3.87 (s, 6H, -OCH3), 4.54 (s, 4H, CH2Br), 6.87 (s, 2H, aromatic H). ¹³C NMR (400MHz, CDCl₃); δ 28.8 (CH₂), 56.5 (OCH₃), 114.0 (C-3,C-6), 127.6 (C-1, C-4), 151.5 (C-2, C-5).

### Triethyl [2,5-dimethoxy-1,4-phenylenebis(methylene)diphosphonate

A solution of 1,4-Bis(bromomethyl)-2,5-dimethylbenzene (10.9 g, 34 mol) and triethylphosphite (13.3 g, 80 mol) was refluxed for five hours at 170°C under nitrogen. After which the excess triethylphosphite was removed under vacuum. The product crystallized from the residue as colorless crystals (11.10 g, 75 %). ¹H NMR (400 MHz, CDCl₃); δ 1.16 (t, 12H, -CH₃), 3.14 (d, *J* = 20.4 Hz, 4H, CH₂P), 3.72 (s, 6H, -OCH3), 3.94 (m, 8H, -OCH₂), 6.83 (d, *J* = 1.2 Hz, 2H, aromatic H). ¹³C NMR (400MHz, CDCl₃); δ 16.5, 26.3, 56.3, 62.0, 114.2, 119.6, 151.2.

### 1,4-Dimethoxy-2,5-bis-[2-(4-nitrophenyl)vinyl]benzene

Triethyl [2,5-dimethoxy-1,4-phenylenebis(methylene)diphosphonate (2.95 g, 6.7 mmol) was dissolved in hydrous THF under an argon atmosphere and potassium *tert*-butoxide (1.50 g, 13.4 mmol) was added to the solution at room temperature. After stirring for 10 min, a solution of 4-nitrobenzaldhyde (2.03 g, 13.4 mmol) in anhydrous THF (30 mL) was added dropwise to the reaction mixture. The reaction mixture was stirred for an additional 4 h at room temperature and subsequently poured into a mixture of crushed ice containing 30 mL of HCl (6mol/L). The mixture was extracted several times with CHCl₃, the organic portions were washed with HCl (3mol/L), water, brine and dried with anhydrous Na₂SO₄. Evaporation of the solvent gave 1.97 g as deep red powder (68% yield). ¹H NMR (400 MHz, CDCl₃); 3.97 (s, 6H, -OCH₃), 7.16 (s, 2H, aromatic H), 7.20 (d, J = 16.4 Hz, 2H, vinylic H), 7.66 (d, J = 16.4 Hz, 2H, vinylic H), 7.68 (d, J = 8.8 Hz, 4H, aromatic H), 8.23 (d, J = 8.8 Hz, 4H, aromatic H). ¹³C NMR (400MHz, CDCl₃); δ 56.5, 109.5, 124.4, 127.2, 127.4, 127.9, 145.9, 148.9, 154.4. HRMS (M⁺), m/z; 432.4930.

### 4-(4-(4-Aminostyryl)-2,5-dimethoxystyryl)benzenamine

Under an argon atmosphere, the nitro compound (1.50 g, 3.47 mmol) was suspended in a mixture of 20 mL ethanol and 5 mL ethyl acetate. Stannous chloride dehydrate (3.91 g, 17.36 mmol) was added to the suspension. The reaction mixture was stirred for 7 h at 80°C, subsequently cooled to room temperature and poured into crushed ice. The aqueous phase was slightly basified by the addition of 0.1M NaOH and extracted several times with diethyl ether. The organic extracts were washed with water, dried over anhydrous sodium sulfate, and solvent was removed in vacuo. Purification by column chromatography (silica gel, pentane/ethyl acetate 2:1 v/v), yielded 0.61 g (47 %) as a dark solid. ¹H NMR (400MHz, CDCl₃); δ 3.73 (br, s, 4H, NH2), 3.90 (s, 6H, -OCH3), 6.67 (d, J = 8.4 Hz, 4H, aromatic H), 7.01 (d, J = 16.4 Hz, 2H, vinylic), 7.09 (s, 2H, aromatic H), 7.29 (d, J = 16.4 Hz, 2H, vinylic H), 7.37 (d, J = 8.4 Hz, 4H aromatic H). ¹³C NMR (400MHz, CDCl₃); δ 56.6 (OCH3), 109.0, 115.4, 119.7, 126.7, 128.0, 128.9, 146.2, 151.5. HRMS (M⁺), m/z; 373.4033.

### 4-(4-(4-(Methylamino)styryl)-2,5-dimethoxystyryl)benzenamine

To a solution of the amine (130 mg, 0.35 mmol) in 5 mL DMF in a reaction vial under an argon atmosphere was added anhydrous potassium carbonate (0.08 g, mmol) and methyl iodide (0.2 mL .35 mmol). The reaction vial was sealed and the mixture stirred for overnight at 100°C. The reaction was cooled to room temperature and diluted with ethyl acetate, washed with water, saturated sodium chloride solution, dried over anhydrous sodium sulfate and solvent removed in vacuo. Purification by column chromatography (silica gel, pentane/ethyl acetate 3:1) yielded 78 mg (58 %) as dark red solid. ¹H NMR (400MHz, CDCl₃); 2.80 (s, 3H, N-CH₃), 3.75 (br, s, 3H, NH, NH₂), 3.90 (s, 6H, -OCH₃), 6.59 (d, J = 8.8 Hz, 2H, aromatic H), 6.66 (d, J = 8.8 Hz, 2H, aromatic H), 7.02 (d, d, J = 6.0, 16.8 Hz, 2H, vinylic H), 7.09 (s, 2H, aromatic H), 7.28 (d,d, J = 7.6, 16.8 Hz, 2H, vinylic H), 7.34 (d, J = 8.4 Hz, 2H, aromatic H), 7.40 (d, J = 8.4Hz, 2H, aromatic H). ¹³C NMR (400MHz, CDCl₃); δ 30.9, 56.8, 108.9, 109.0, 112.7, 115.4, 119.1, 119.9, 126.5, 127.0, 127.5, 128.0, 128.8, 129.0, 129.1, 146.2, 149.1, 151.5. HRMS (M⁺), m/z; 387.4524.

### C-11Radiolabelling of CIC.

High specific radioactivity [¹¹C] methyl iodide was synthesized from [¹¹C] carbon dioxide utilizing a homemade one-pot reaction apparatus. Briefly, [¹¹C] carbon dioxide was produced by scanditronix MC17 cyclotron (10 min bombardment) and bubbled into a reaction device previously filled with LiAlH₄ in tetrahydrofurane (THF) solution (0.1 mol/L, 1ml) at room temperature. After THF was completely evaporated by argon gas, hydriodic acid (HI, 57%, 1ml) was then added and the device was heated to 120. [¹¹C] methyl iodide (3700MBq) was distilled and trapped into a reaction vial containing the precursor BDB (2mg), K₂CO₃ (10mg) and DMF (0.3ml) at -70° (dry ice) for 10 min. The reaction vial was then heated to 140° for another 10 minutes and quenched with 10ml of H₂O and cooled to room temperature. The resulting reaction mixture was loaded onto a Sep-Pak C-18 column and followed by washing with 10 ml of H₂O and rapid air bolus. The final product ¹¹C-CIC was eluted by 2 ml of ethanol and purified by HPLC (Phenomenex, C18, 250 × 10mm, CH₃CN / H₂O = 80/20 (V/V), flow rate 3ml/min). The desired fractions were collected and evaporated to dryness under argon to give the final product ¹¹C-CIC (296MBq). For injection, the residue was dissolved in 5% DMSO in saline and filtered through a 0.2 µm sterile membrane filter (Millex-GS Filter, Fisher) into a sterile vial. The totally synthesis time was about 40 min (from ¹¹CH₃I), the radiolabeling yield was about 32% (decay corrected to ¹¹CH₃I) and radiochemical purity was over 98% determined by radio-HPLC system. The chemical identity was verified by co-injection of the cold standard.

### Measurement of partition coefficients (logPoct)

Partition coefficients was measured by mixing the radioligands (radiochemical purity was greater than 98%, approximately 500,000 cpm) with 1-octanol (3 g, 3.65 mL) and sodium phosphate buffer (PBS, 0.1 mol/L, 3 g, pH 7.40) in a test tube. After consistent partitions of the coefficient values were obtained, the partition coefficient was determined by calculating the ratio of cpm/g of n-octanol to that of PBS. All assays were performed in triplicate.

### Brain uptake studies

Mice (Swiss Webster, 22 ∼ 24g) received 2 ∼ 22 MBq of high specific activity [¹¹C] CIC in 0.2 ml of buffered saline via the tail vein. The mice were sacrificed by decapitation at various time intervals at 5, 30 and 60 min postinjection. The brains were removed and collected, weighed and counted. Radioactivity in tissue was assayed in an automated gamma counter, decay corrected to time of injection. The uptake of brain and blood were expressed in percentage injected dose (%ID) per gram organ. The percentage injected dose per gram organ of sample was calculated by comparing the sample counts with the count of the diluted initial dose.

### Focal demyelination of adult rat brain

Twelve-week old, female Sprague-Dawley rats were deeply anesthetized using an intramuscular cocktail of Ketamine hydrochloride (64.2 mg/kg), Xylazine hydrochloride (12.9 mg/kg), and Acepromazine (2.1 mg/kg). Animals were then secured in a Stoelting stereotactic apparatus. Using a #10 scalpel blade, a midline scalp incision was made to expose bregma. Using published coordinates for the corpus callosum, striatum, and cerebellum, burr holes were drilled into the skull for access to brain parenchyma. To induce demyelination, 6 µl lysolecithin (Sigma) were then injected at a rate of 0.50 µl/minute using a 10 µl Hamilton syringe connected to a Stoelting microinjection system. Incisions were closed using Ethicon 5.0 sutures. Post-operatively, animals received a subcutaneous injection of 5 ml saline to ensure adequate hydration. Animals were allowed to recover on a heating pad and sacrificed at various times post-lesion.

### MRI studies

All MRI imaging experiments were performed on a Bruker Biospec 7.0T/30cm Magnetic Resonance Imaging and Spectroscopy scanner in the Case Center for Imaging Research at Case Western Reserve University (Bruker Biospin, Billerica, MA). The animal's head was then positioned in a 72-mm volume ID cylindrical transceiver coil. A RARE acquisition (TR/TE=2000/40ms, 4 echoes, FOV=45mmx45mm, matrix=256x256) was used to acquire 15 contiguous 1-mm axial images of each animals brain. The lesion area in each image was measured by selecting the conspicuous lesion ROIs.

MicroPET studies Animals were placed in a Concord R4 microPET scanner (Knoxville, TN) under anesthesia. After a 10 min transmission scan with a Co-57 source, 2 mCi/kg of radiolabelled agents were administered to each animal through a tail vein injection, which was immediately followed by dynamic acquisition for up to 90 min.

List-mode emission data was analyzed as histograms with 12 x 5-sec, 12 x 30-sec, 5 x 60-sec, and 17 x 300-sec dynamic frames. A 2-D filtered back projection (FBP) algorithm was used for image reconstruction with a 256x256-pixel resolution per transverse slice. A total of 63 transverse slices were reconstructed with a field of view covering the brain regions. Decay correction, attenuation correction and scatter correction were all performed during the image histogram and reconstruction processes.

### Co-registration of Images and Statistical Analysis

Coregistration of MRI and PET images were conducted by using the MATLAB-based program COMKAT (Compartmental Model Kinetic Analysis Tool). The registration was conducted using a coronal view of the rats. After creating uniformed images from the PET and MRI images, VOI (Volume of interest) and ROI (Region of interest) were defined and used to measure the radioactivity concentration on one side of the corpus callosum (demyelinated) and its mirror counterpart on the other side of the corpus callosum (non-demyelinated). Multiple time activity curves were then obtained for statistical analysis. The radioactivity data were decay-corrected and normalized by the body weight of the rats and amount of [¹¹C]CIC injected. The resulting normalized time activity curves obtained from different data sets were averaged to obtain a representative normalized time activity curve for the experiments. A student t-test was used to evaluate if there was any significant difference between the curves. A ρ value < 0.05 was accepted as significant.

### Chemistry

The reaction steps depicted below provides the synthetic entry into the CIC, [¹¹C]CIC, and its precursor. 1,4-dimethoxybenzene was bisbromomethylated in glacial acetic acid solution with paraformaldehyde and hydrogen bromide to provide a 1,4-bis(bromomethyl)-2,5-dimethoxybenzene 2 in 90 % yield. The two bromomethylenes were confirmed to be at position 2- and 5-positions, by both ¹H- and ¹³C-nuclear magnetic resonance spectra.

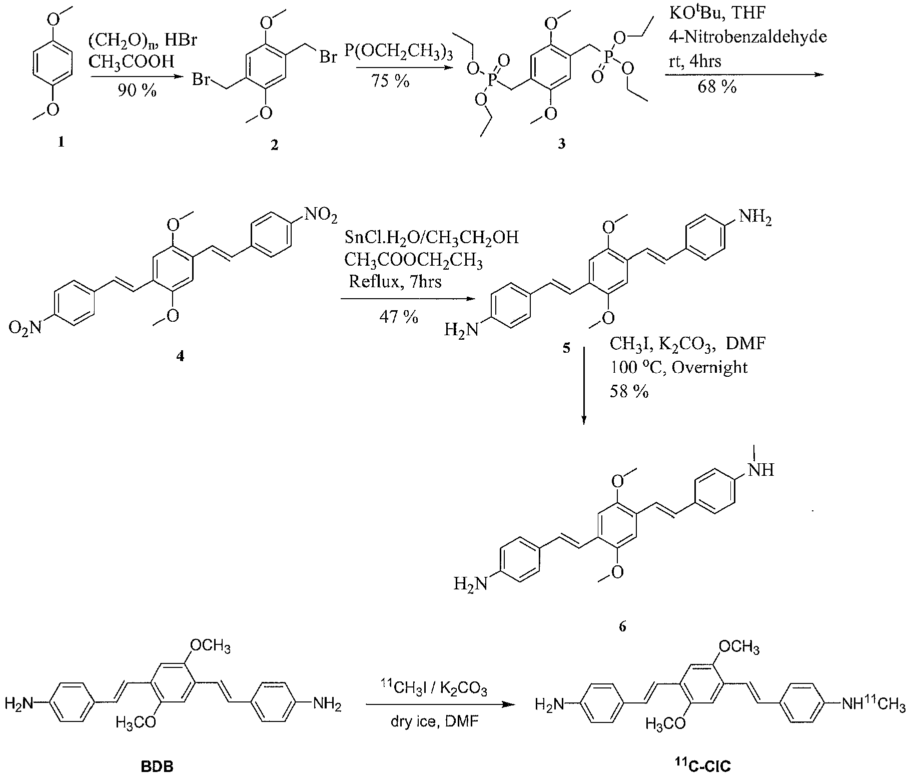

The intermediate diphosphonate, 3 was synthesized from the 1,4-bis(bromomethyl)-2,5-dimethoxybenzene, 2 according to literature precedent in 75 % yield (26-29). Coupling of the diphosphonate, 3 with two equivalent of 4-nitrobenzyaldehyde under Wittig-Horner-Emmons coupling condition using sodium hydride as a base in THF for 4hrs at room temperature gave the dinitro-derivative product, 4 (68 % yield). The *trans* stereochemistry of the olefin linkages was established by the coupling constant of the vinylic protons in the ¹H-NMR spectra (*J* ≈ 16 Hz). The ¹H-NMR spectra further displayed a conjugated pattern: two doublets signals for the outer phenyl rings and vinyl protons respectively and singlet signals for the central phenyl system. The synthesis of the diamine derivatives was fairly straightforward, reduction of the dinitro-derivative, 4 with stannous chloride dehydrate gave the expected diamine derivative, 5 in 43% yield(31, 32). Finally the reaction of diamine 5 with one equivalent of methyl iodide in DMF at 100°C, gave the expected product compound 6 in 58%.

The synthesis of the target [¹¹C]CIC was accomplished using 5 in dimethyl formamide with [¹¹C]methyl iodide in the presence of potassium carbonate as outlined in Scheme 2. The cyclotron-derived [¹¹C]carbon dioxide was converted to [¹¹C]methyl iodide by reduction with lithium aluminum hydride and hydroiodic acid. The labeled methyl iodide ([¹¹C]CH₃I) formed was concurrently distilled and trapped in a DMF solution containing 5 and potassium carbonate. Trapping was monitored by the isotope calibrator until the maximal value was attained. Methylation reaction was carried out at 140°C for 10 min and the resulting mixture was subjected to a prepurification procedure using (solid-phase extraction) prior to sempreparative HPLC purification. The decay-corrected radiochemical yield of the product [¹¹C]CIC obtained after preparative HPLC purification was >20 % base on the activity of [¹¹C]methyl iodide trapped. Identification of the product and chemical radiochemical purities were determined by the co-injection with the reference standard 6 in radioactive HPLC chromatogram and they showed the same retention time peaks on the UV and the radioactive chromatograms. The radiochemical purity of [¹¹C]CIC was > 95%. The retention time of the product [¹¹C]CIC on the analytical HPLC was approximately 9.0 min and the synthesis was completed in 40-50 min including purification (preparation of the methylating agent is not included)

### In vitro studies

Lipophilicity Following synthesis and radiolabelling, the lipophilicity of [¹¹C]CIC was determined in terms of partition coefficients (logPoct). Lipophilicity is an important factor in brain permeability. Previous study has shown that small-molecule compounds with a logPoct value ranging from 1.5-3.5 normally exhibit optimal brain upake. Using the conventional octanol-water partition method, we determined the logPoct value of [¹¹C] CIC as 2.36 ± 0.22 (n=3). This value falls into the range to exhibit optimal brain permeability.

### In vitro tissue staining of mice

CIC is a fluorescent compound. The maximal excitation and emission of CIC is 426 nm and 506 nm, respectively. This allows us to evaluate its staining properties based on fluorescent microscopy.

We first examined the white matter binding property of CIC with fluorescent microscopy of mouse brain tissue sections. Following the preparation of frozen sections of wild-type mice, fluorescent tissue staining was conducted with CIC and, for comparison, black gold staining of myelin was performed in the same sections. Sagittal sections were used so that both regions rich and poor in myelin could be easily compared. At a 10 µM concentration, CIC selectively labeled myelinated white matter regions such as the corpus callosum (Fig. 9, upper panel) and cerebellum (Fig. 9, lower panel). The staining pattern using CIC was found identical to the pattern detected by black gold staining. In a sharp contrast, gray matter regions including those in the cerebellum and the frontal cortex showed little if any CIC staining. These studies demonstrate that BDB selectively labels white matter.

### In vitro tissue staining of focal demyelination in rats

Encouraged by the selective localization of CIC in myelinated white matter regions in the wild-type mouse, we tested the possibility that CIC would be capable of detecting demyelinated lesions. We then conducted CIC staining of tissue sections prepared from a rat model of focal demyelination (Ref). In this rat model, demyelinated lesions were induced by treatment with lysolecthin to induce demyelinated lesions. The presence of demyelinated foci was first confirmed by blackgold staining for myelin tracts (Fig. 10A). The same foci were then detected by fluorescent staining of serial sections with BMB (Fig. 10B), which was superimposed with black gold staining (Fig. 10C).

### In Situ Staining of Myelin

### Brain Uptake Studies in Mice

CIC readily enters the mouse brain. Following a single tail vein injection of ¹¹C-CIC, the radioactivity concentration of [¹¹C]CIC in the brain was determined at 5, 30 and 60 min post injection. As shown in Fig. 11, at 5 minutes after injection, the brain uptake reached 1.83 ± 0.22%ID/g. At 30 minutes, the brain concentration decreased to 2.30 ± 0.34% ID/g, and at 60 minutes, the brain concentration remained fairly steady (2.35 ± 0.30%ID/g, close to the 30 minute value).

### In Situ tissue staining

Following *in vitro* studies and brain uptake in rodents, we investigated the ability of CIC to stain myelinated white matter *in situ* in the mouse brain. A dose of 1.0 mg CIC (50 mg/kg) was injected via the tail vein into wild-type mice. Eighteen hours post injection, mice were perfused with saline to clear the blood vessels in the brain, and the brains were then removed and sectioned. CIC staining of myelin was then directly examined under fluorescent microscopy. As shown in Fig. 12, CIC entered the brain and selectively labeled the corpus callosum (Fig. 12A) and cerebellum (Fig. 12B) of the wild-type mice. Subsequent immuno-staining for MBP revealed that BDB bound more selectively to white matter regions containing myelin fibers.

### In Vivo microPET Imaging

Following the above *in vitro* and *ex vivo* studies, we conducted microPET in order to directly quantify demyelination *in vivo* in animal models. The goal is to assess and validate the myelin-imaging agent in animal models of demyelination. Our microPET scanner (Concord R4) performs with approximately 2x2x2 mm image resolution (FWHM), resulting in a volumetric resolution of ∼8 mm³. The size of demyelinated lesions varies, but can be induced at 5 mm in diameter on average. Therefore, microPET is capable of detecting and quantifying myelin changes in animal models. When demyelinated lesions are 2 mm or less, we would encounter the issue of resolution limitation of microPET. To address this issue, we first conducted high resolution of MR imaging. High resolution MR images with a typical in-plane resolution of 100 µm will allow for accurate detection of demyelinated lesions in order to define regions of interest. The microPET images are then registered with MR images so that radioactivity concentration can be quantified based on the regions of interest drawn on MR images. Co-registration of MRI and microPET images is a widely used technique to overcome the resolution limitation of microPET.

In this study, we used a rat model with focal, reversible central nerve demyelination for microPET imaging studies to take advantage the relatively large size of the brain. In this model, we used the selective demyelinating agent lysolecithin (lysophosphatidyl chorine) to induce demyelinated lesions in the corpus callosum of one hemisphere of the rat brain.

Five days after lysolecithin treatment, demyelinated lesions up to 5 mm in diameter were readily detected by high resolution 7T MRI. The lesions were defined as the regions of interest (ROI) and radioactivity concentrations were quantified. For comparison, the symmetric region of the same size in the other hemisphere of the rat brain without demyelination were selected and used as reference. We then conducted microPET imaging of the rat brain using a C-11-labeled CIC. The resultant microPET images were registered to the MRI images, which allowed us to quantify the radioactivity concentrations in the demyelinated regions.

As shown in Fig. 13-16, the radioactivity concentrations were determined as standard uptake value in each region of interest. Compared to the normal myelinated regions, the demyelinated regions of the same rat brain showed significantly lower radioactivity retention throughout the 60 min of PET scan. In the corpus callosum, the average uptake in the demyelinated region was 17% lower than that in the non-myelinated reference region. In the cerebellum, the average uptake in the demyelinated region was 28% lower than that in the non-demyelinated reference region. The difference of uptake between the two regions can be improved through structural optimization.

Nonetheless, these studies clearly demonstrated the feasibility of using microPET to quantify demyelination *in vivo* in animal models. Following imaging studies, all of the animals survived and showed no signs of any behavioral changes, indicating the radioligands have no apparent toxicity.

## Claims

1. A molecular probe for use in the *in vivo* detection by positron emission tomography of myelination in a subject comprising fluorescent stilbenzene derivative that is less than 700 daltons and has a binding affinity (Kd) to isolated myelin fractions of at least about 100nM and a binding affinity (Kd) of up to about 10µM to isolated non-myelin fractions, the fluorescent stilbenzene derivative having the formula

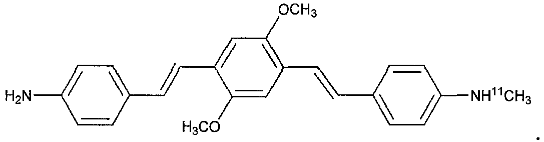

2. Use of the molecular probe of claim 1 in a method of detecting myelination *in vivo* in brain tissue of a subject.

3. Molecular probe of claim 1 for use in the *in vivo* diagnosis by positron emission tomography of a myelin related disorder in the subject.

4. Use of the molecular probe of claim 1 in a method of monitoring the efficacy of remyelination therapy in a subject.

5. Use of the molecular probe of claim 1 in a method of screening the myelination effects of an agent in brain tissue of the subject.

## Patentansprüche

1. Molekulare Sonde zur Verwendung in dem *in vivo* Nachweis von Myelination mit Positronenemissionstomographie bei einem Probanden umfassend fluoreszierendes Stilbenzenderivativ, das weniger als 700 Daltons und eine Bindungsaffinität (Kd) zu isolierten Myelinfraktionen von mindestens ungefähr 100nM und eine Bindungsaffinität (Kd) von bis zu ungefähr 10µM zu isolierte Nicht-Myelinfraktionen aufweist, wobei das fluoreszierende Stilbenzenderivativ die Formel 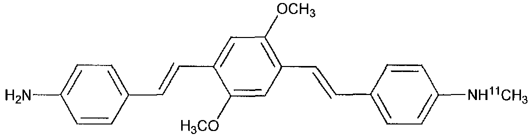 hat.

2. Verwendung der molekularen Sonde gemäß Anspruch 1 in einem Verfahren zum Nachweis von Myelination *in vivo* in Gehirngewebe von einem Probanden.

3. Molekulare Sonde gemäß Anspruch 1 zur Verwendung in *in vivo* Diagnose mit Positronenemissionstomographie von einer Myelin-assoziierten Störung in einem Probanden.

4. Verwendung einer molekularen Sonde gemäß Anspruch 1 in einem Verfahren zur Überwachung der Effizienz einer Remyelinierungstherapie in einem Probanden.

5. Verwendung der molekularen Sonde gemäß Anspruch 1 in einem Verfahren zum Screening von Myelinierungseffekten von einem Agens in Gehirngewebe in einem Probanden.

## Revendications

1. Sonde moléculaire pour une utilisation dans la détection *in vivo* par tomographie par émission de positrons de la myélinisation chez un sujet comprenant un dérivé de stilbenzène fluorescent qui est inférieur à 700 daltons et présente une affinité de liaison (Kd) à des fractions de myéline isolées d'au moins environ 100 nM et une affinité de liaison (Kd) de jusqu'à environ 10 µM à des fractions non-myéline isolées, le dérivé de stilbenzène fluorescent ayant la formule

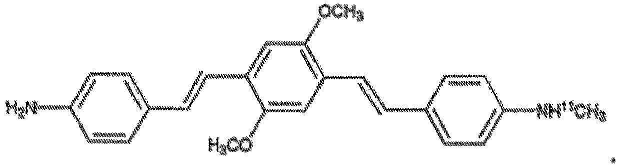

2. Utilisation de la sonde moléculaire selon la revendication 1 dans un procédé de détection de la myélinisation *in vivo* dans du tissu cérébral d'un sujet.

3. Sonde moléculaire selon la revendication 1 pour une utilisation dans le diagnostic *in vivo* par tomographie par émission de positrons d'un trouble associé à la myéline chez le sujet.

4. Utilisation de la sonde moléculaire selon la revendication 1 dans un procédé de surveillance de l'efficacité d'un traitement de remyélinisation chez un sujet.

5. Utilisation de la sonde moléculaire selon la revendication 1 dans un procédé de criblage des effets de myélinisation d'un agent dans du tissu cérébral du sujet.
